(19) Europäisches Patentamt
European Patent Office
Office européen des brevets

(11) **EP 2 151 219 B1**

(12) **EUROPEAN PATENT SPECIFICATION**

(45) Date of publication and mention
of the grant of the patent:
**22.10.2014 Bulletin 2014/43**

(51) Int Cl.:
**A61F 5/441** (2006.01)     **A61F 5/445** (2006.01)
**A61L 9/00** (2006.01)

(21) Application number: **08740878.7**

(86) International application number:
**PCT/JP2008/058077**

(22) Date of filing: **25.04.2008**

(87) International publication number:
**WO 2008/136413 (13.11.2008 Gazette 2008/46)**

(54) **DEODORIZING FILTER AND EXCRETA RECEPTACLE HAVING THE SAME**

DESODORISIERENDER FILTER UND DAMIT AUSGESTATTETER BEHÄLTER ZUR AUFNAHME
VON EXKREMENTEN

FILTRE DÉSODORISANT ET RÉCEPTACLE POUR EXCRETA LE COMPORTANT

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **02.05.2007   JP 2007121399**

(43) Date of publication of application:
**10.02.2010   Bulletin 2010/06**

(73) Proprietor: **Alcare Co., Ltd.**
**Sumida-ku**
**Tokyo 131-0046 (JP)**

(72) Inventor: **KAMBARA, Noriyuki**
**Tokyo 131-0046 (JP)**

(74) Representative: **Kahlhöfer, Hermann**
**KNH Patentanwälte**
**Kahlhöfer Neumann Rößler Heine**
**Postfach 10 33 63**
**40024 Düsseldorf (DE)**

(56) References cited:
**EP-A2- 1 051 955         DE-A1- 4 441 589**
**GB-A- 2 059 797          GB-A- 2 276 324**
**JP-A- 2003 010 226       JP-A- 2005 520 581**
**JP-B2- 3 590 084         US-A- 4 318 406**
**US-A- 5 306 264          US-A- 5 468 235**

## Description

TECHNICAL FIELD

The present invention relates to a deodorizing filter and an excrements collector provided with the deodorizing filter. More particularly, the invention relates to a deodorizing filter for use in an excrements collector, which is fitted around the aperture or the wound formed in the body surface to thereby temporarily hold the excretory substances, excretory fluids, gases, exudates, secretory fluids and others (hereinafter these are referred to as a generic term "excrements") from inside the living body or from the surface thereof, and to an excrements collector provided with the deodorizing filter.

A deodorizing filter according to the preamble of claim 1 is disclosed in US-A-5 306 264.

BACKGROUND ART

In case where a person could not control the excretion of feces and urine on a voluntary basis or in case where a person has a digestive system or urinary tract system disease, the person may undergo a surgical operation so as to lead the intestinal tract or the urinary tract thereof out as far as the body surface, and an aperture called a stoma may be formed in the body surface, and the person must wear an ostomy pouch that can temporarily hold therein the excrements from the stoma, around the stoma.

A person who has an aperture or a wound in the body surface owing to any other disease must also wear a drainage pouch for disposing the excrements discharged from a drainage or the like, around the aperture or the wound.

These excrements collector such as an ostomy pouch, a drainage pouch and the like generally comprise a faceplat and a pouch, in which the faceplate is fixedly adhered to the portion around the stoma or the like in the body surface by an adhesive layer of the faceplate and has the function of guiding the excrements into the pouch without allowing the leakage of the excrements.

In the excrements, gases with odors are contained in some cases; and, as the excrements are discharged, the gases also more and more increases in the pouch, so that there is the risk that the pouch may finally be ruptured unless the gases are suitably vented. Once the pouch is ruptured, the excrements leak out. To evade the situation, a deodorizing filter is disposed in a part of the pouch, and the gas discharged inside the pouch is diffused out of the pouch while the odor thereof is removed through the deodorizing filter.

In this case, when the deodorizing element of the deodorizing filter is disposed as exposed out inside the pouch so as to be in direct contact with excrements, then the excrements may adhere to the surface of the deodorizing element to clog the filter. As a result, the gas venting function may worsen, and the excrements may penetrate into the deodorizing element and may at last leak outside.

To solve this problem. Patent Reference 1 proposes providing a liquid-impervious and vapor-pervious film, or that is, a layer that is pervious to vapor but is impervious to liquid (hereinafter referred to as a breathable waterproof layer) on the surface of a deodorizing element to thereby secure good vapor penetration and insulation of the filter from contact with excrements. The breathable waterproof layer is generally hydrophobic and therefore can prevent moisture in excrements from penetrating and adhering thereto.

However, excrements may contain substances having a hydrophobic moiety in the chemical structure thereof such as protein, lipid and the like, and these substances may readily adhere to the surface of the breathable waterproof layer and may gradually clog it, therefore resulting in gas venting failure. Owing to the clogging of the breathable waterproof layer, the user must often exchange the excrements collector earlier than the period acceptable for ordinary use, which is therefore not only uneconomical but also may cause a skin trouble owing to the peeling irritation resulting from such frequent collector exchange.

This phenomenon is often seen in ileostomy of leading an ileum out as far as the body surface for forming aperture therein. The feces in ileostomy are liquid or muddy, and may often contain lipid therein, and therefore they may often penetrate into and adhere to the pores in the surface of the breathable waterproof layer thereby clogging them.

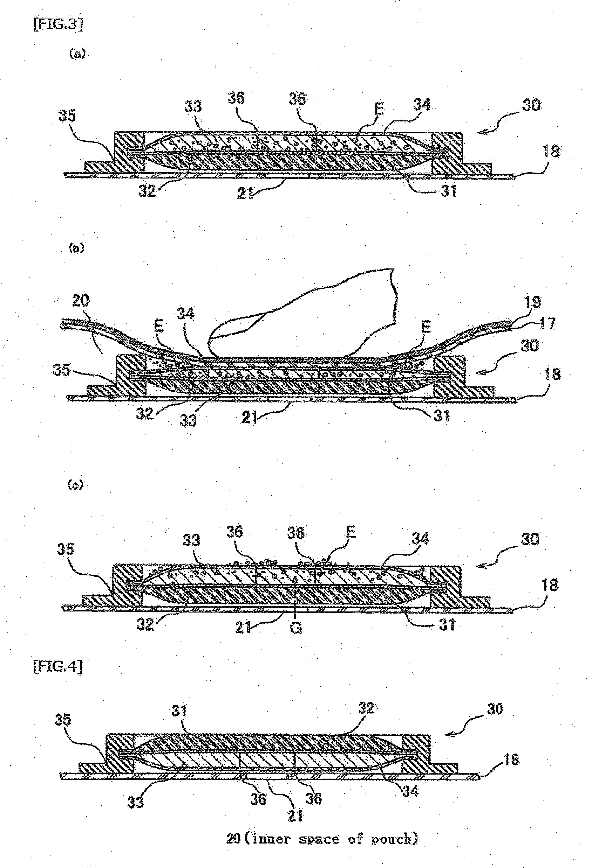

Fig. 3 to Fig. 6 in Patent Reference 2 disclose a multistage filter comprising, as sequentially superimposed one upon another, a deodorizing filter containing activated carbon, a porous film having a large number of micropores, a protective filter formed of an open-cell polyurethane foam and a liquid-impervious film wafer formed of polyethylene, in the given order. The multistage filter is so constituted that the peripheral parts of the individual layers superimposed one upon another are separately adhered to the wall of an ostomy bag so as to concentrically surround the deodorizing filter. In the multistage filter, the discharged gas sequentially passes through the constitutive layers, and the gas penetrates from the periphery of each layer to flow toward the center, and is finally discharged out of the ostomy bag.

The multistage filter in Patent Reference 2 has a protective filter formed of an open-cell polyurethane foam and therefore can prevent penetration of semiliquid excrements thereinto; and as compared with conventional ones, clogging the filter may be delayed. However, even the protective filter existing therein could not completely prevent the penetration of excrements into inside of the filter; and therefore, in long-term use of the bag, the excrements may gradually move toward the porous film, and at last, there may occur a problem of clogging of the pours of the porous film therefore resulting in gas venting failure.

According to the conventional art as above, only multistage-structured filters or the like have been planned on the basis of the idea of prolonging as much as possible the time to be taken by the excrements to reach the breathable waterproof layer; however, there is known no technology of evading penetration and adhesion of excrements to the breathable waterproof layer and making it possible to readily remove the adhered excrements, if any in long-term use, from the breathable waterproof layer to thereby solve the problem of clogging of the breathable waterproof layer.

Patent Reference 1: JP-A 4-218156
Patent Reference 2: Japanese Patent 3590084

DISCLOSURE OF THE INVENTION

PROBLEMS THAT THE INVENTION IS TO SOLVE

The present invention has been made in consideration of the above-mentioned problems, and the subject matter of the invention is to provide a deodorizing filter that even in long-term use, is free from clogging by excrements and can maintain a favorable gas venting function, and to provide an excrements collector having the deodorizing filter.

MEANS FOR SOLVING THE PROBLEMS

To solve the above-mentioned problems, the deodorizing filter of the invention comprises the features of claim 1.

In the deodorizing filter, the peripheral parts of the laminate are sealed up so as to protect the peripheral parts of the laminate from gas penetration thereinto. Further, in the deodorizing filter, the peripheral parts of the three layers of the breathable waterproof layer, the porous material layer and the cover layer are sealed up all together. In the deodorizing filter, the peripheral parts of the three layers of the breathable waterproof layer, the porous material layer and the cover layer are sealed up all together under compression, and the surface of the cover layer forms a curved surface. Further, in the deodorizing filter, the laminate undergoes elastic deformation when its surface is pressed.

The deodorizing filter comprises a laminate of, as sequentially superimposed one upon another, a deodorizing layer, a breathable waterproof layer covering a one-side surface of the deodorizing layer, a porous material layer covering a one-side surface of the breathable waterproof layer and a cover layer covering a one-side surface of the porous material layer and having through-holes, wherein the peripheral parts of the laminate are sealed up all together under compression so as to protect the laminate from gas penetration through the peripheral parts thereof and the surface of the cover layer forms a curved surface, and wherein the laminate undergoes elastic deformation when its surface is pressed, and gas may pass through the through-holes of the cover layer, the porous material layer, the breathable waterproof layer and the deodorizing layer in the given order.

As embodiments of the invention, the following are preferred. Specifically, in the deodorizing filter, the peripheral parts of the laminate are surrounded by a frame that is harder than the laminate. In the deodorizing filter, the porous material layer is formed of an open-cell foam or a three-dimensional spatial fabric. In the deodorizing filter, the through-holes of the cover layer are slit-like ones. In the deodorizing filter where the through-holes of the cover layer are slit-like ones, the slit-like through-holes of the cover layer are so formed as to be adjacent to the peripheral part of the cover layer.

Of the various constitutive layers of the laminate, each "layer covering a one-side surface" is preferably the "layer covering at least 80% of the area of the one-side surface", more preferably covering substantially the entire area thereof. Elastic deformation is meant to indicate the property of a substance which, when its shape or volume has changed owing to an external force given thereto, can be restored to its original condition after removal of the force.

The excrements collector of the invention comprises the features of claim 7.

ADVANTAGE OF THE INVENTION

According to the invention, the cover layer and the porous material layer may prevent the adhesion of excrements to the breathable waterproof layer for a relatively long period of time. Even though excrements may penetrate near to the breathable waterproof layer of the deodorizing filter, the excrements could be readily led out of the deodorizing filter when the laminate of the deodorizing filter is pressed, because of the predetermined laminate structure. Since the deodorizing filter has the predetermined laminate, the laminate may readily undergo elastic restoration and the porous material layer may take a fluid (air and excrements) suction effect, and therefore the excrements adhering near to the breathable waterproof layer can be removed and diffused thereby solving the problem of clogging the breathable waterproof layer.

In the manner as above, the deodorizing filter can remove repeatedly many times excrements that cause clogging owing to the effect thereof of discharging and sucking excrements, and therefore even in long-term use thereof, the filter can keep its good gas venting function.

BRIEF DESCRIPTION OF THE DRAWINGS

[Fig. 1] It shows a first embodiment of the excrements collector.
[Fig. 2] It shows enlarged views of the deodorizing filter part of the first embodiment.
[Fig. 3] It explains the effect of the deodorizing filter.
[Fig. 4] It shows a second embodiment of the excrements collector.

DESCRIPTION OF REFERENCE NUMERALS AND SIGNS

| 10 | Excrements Collector |
| 11 | Faceplate |
| 12 | Pouch |
| 13 | Opening |
| 21 | Gas-Discharging Vent Hole |
| 30 | Deodorizing Filter |
| 31 | Deodorizing Layer |
| 32 | Breathable Waterproof Layer |
| 33 | Porous Material Layer |
| 34 | Cover Layer |
| 35 | Frame |
| 36 | Through-hole |
| E | Excrements |
| G | Air |

BEST MODE FOR CARRYING OUT THE INVENTION

Next, embodiments of the invention are described with reference to the drawings. The same reference numeral or sign is given to the same part for its description.

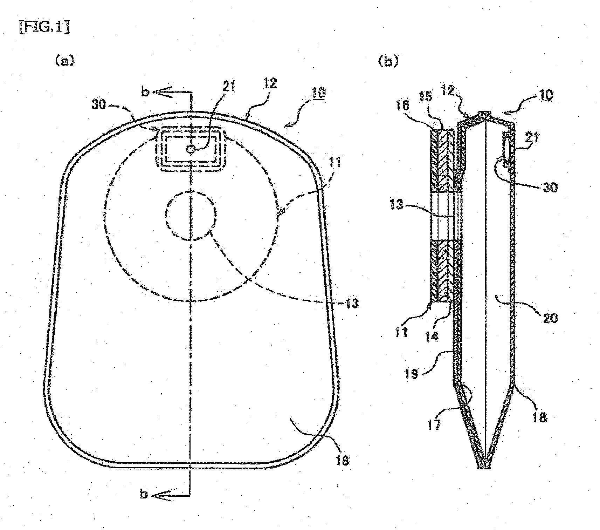

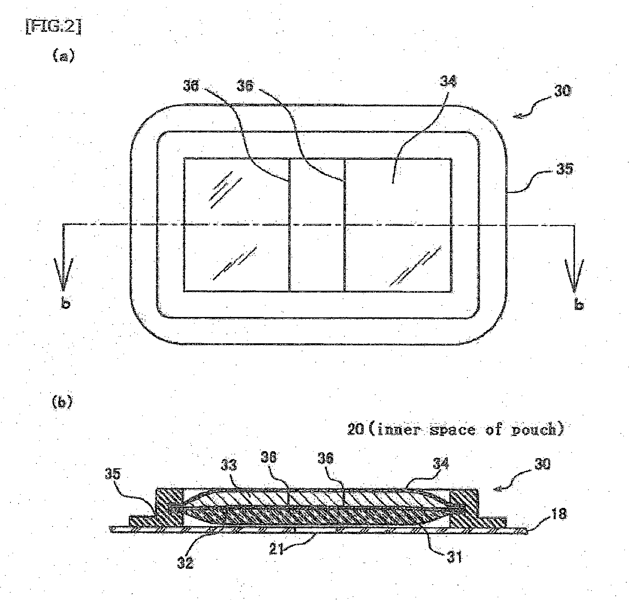

Fig. 1 and Fig. 2 show a first embodiment of the excrements collector having a deodorizing filter.

In Fig. 1, (a) is a front view of the whole, and (b) is a b-b line cross-sectional view of (a).

In Fig. 2, (a) is an enlarged front view of the deodorizing filter part of (a), and (b) is a b-b line cross-sectional view of (a).

The excrements collector 10 comprises a faceplate 11 to adhere and fix around the aperture or the like formed in the surface of a living body, and a pouch 12 to hold the excrements discharged from the aperture or the like, and an opening 13 for receiving excrements and others into the pouch is formed in the faceplate and the pouch to run through them. The faceplate 11 has a support layer 14, an adhesive layer 15 and a peelable adhesive protective layer 16, and in use, the adhesive protective layer 16 is peeled off, and the faceplate is fixed to the body surface via the adhesive layer 15.

The pouch 12 is fixed on the support layer 14 of the faceplate 11 and holds therein the excrements discharged from the aperture in the body surface via the opening 13. The pouch 12 has an inside (body side) film 17 and an outside film 18 (opposite to the body side); and a nonwoven fabric 19 is provided on the skin side of the inside film 17 for preventing the sticky feeling and sweaty state owing to the contact of skin to the inside film 17. The inside film 17, the outside film 18 and the nonwoven fabric 19 are sealed up together, for example, by heat sealing at the outer peripheries thereof to form an inner space 20.

In the upper portion of the outside film 18, provided are a gas-discharging vent hole 21 through which the inner space 20 of the pouch communicates with the outside of the pouch, and a deodorizing filter 30 to cover the gas-discharging vent hole 21.

The deodorizing filter 30 comprises a laminate of, as sequentially superimposed one upon another, a deodorizing layer 31, a breathable waterproof layer 32 covering the whole of a one-side surface of the deodorizing layer, a porous material layer 33 covering the whole of a one-side surface of the breathable waterproof layer and a cover layer 34 covering the whole of a one-side surface of the porous material layer, and is so designed that the peripheral parts of the laminate are surrounded by a frame 35 harder than the laminate. In the first embodiment, the deodorizing filter 30 is mounted on the inner surface of the pouch so that the side of the deodorizing layer 31 could face the gas-discharging vent hole 21.

In the surface of the cover layer 34, formed are slit-like through-holes 36 that the gas staying in the inner space 20 are led to pass through the deodorizing filter 30. In the first embodiment, the slit-like through-holes 36 are so formed as to be adjacent to the peripheral part of the cover layer 34 and reach the porous material layer 33.

The gas staying in the inner space 20 passes through the through-hole 36 of the cover layer 34, then sequentially passes through the porous material layer 33 and the breathable waterproof layer 32 and is thereafter deodorized in the deodorizing layer 31 and discharged out of the pouch via the gas-discharging vent hole 21. During this gas discharging, the peripheral parts of the laminate are all compressed and sealed up with the frame 35, and therefore excrements (gas) do not flow into the laminate through the peripheral parts thereof.

Next described is the effect of the deodorizing filter with reference to Fig. 3. The deodorizing filter shown in Fig. 3 is the first embodiment of the invention, and is the same as that described in Figs. 1 and 2. As in Fig. 3(a), in long-term use of the excrements collector, the excrements E having flown into the deodorizing filter through the through-holes 36 of the cover layer 34 are trapped by the porous material layer 33, and some of them may reach the breathable waterproof layer 32.

In the case of Fig. 3(a), when the laminate of the deodorizing filter is pushed on the top of the pouch as in Fig. 3(b), the laminate is compressed and a part of the excrements E existing in the porous material layer 33 are discharged out through the slit-like through-holes 36 of the cover layer 34. In this condition, when the surface of the cover layer forms a curved surface as convexed in the center thereby to have a curved cross-section profile as in this embodiment, then the slits around the periphery of the cover layer are hardly blocked up and therefore excrements may be readily discharged out through the slits when the laminate is pushed.

Next, when the compression by the fmger is removed as in Fig. 3(c), then the laminate undergoes elastic deformation and is restored to its original form. In this stage, with the elastic restoration of the porous material layer, the excrements existing inside the porous material layer are sucked up toward the cover layer, and further, owing to the air G flowing into the laminate from the side of the breathable waterproof layer thereof, the excrements around the breathable waterproof layer are also removed and diffused. As a result, the breathable waterproof layer 32 is not clogged and can keep a good gas venting function

In the deodorizing filter, the peripheral parts of the laminate are all compressed and sealed up with the frame 35 that is harder than the laminate, and therefore, the layers constituting the laminate are tightly adhered to each other with no space therebetween, and after compression, the laminate readily undergoes elastic restoration. Accordingly, the laminate has a good effect of sucking up excrements.

Next, the components constituting the deodorizing filter 30 of the invention are described in detail.

(Regarding Deodorizing Layer)

The deodorizing layer 31 in the first embodiment is composed of a fibrous activated carbon. As the deodorizing ingredient of the deodorizing layer, also usable are any other deodorizing substances than activated carbon, for example, polyphenol compounds, ester compounds, aldehyde compounds, quaternary ammonium compounds, inorganic metal compounds, enzymes, etc.

Preferably, the deodorizing layer can undergo elastic deformation when pressed with a finger, and can be restored to its original condition; and preferably, the layer is a sheet-like material having a fibrous or open-cell foam substrate.

(Regarding Breathable Waterproof Layer)

The breathable waterproof layer 32 in the first embodiment is composed of a polyamide nonwoven coated with an acrylic copolymer resin and a polytetrafluoroethylene resin. Preferably, the breathable waterproof layer is pervious to vapor but impervious to water, or that is, it has the property of letting air pass through it but not water. More preferably, the layer has a water pressure resistance of at least 800 mmH$_2$O.

.As the breathable waterproof layer, also usable are those prepared by coating the surface of a sheet-like material such as nonwoven fabric, knitted fabric, textile fabric, paper, porous plastic film or the like with a hydrophobic resin. The hydrophobic resin includes, for example, fluororesin, silicone resin, acrylic resin, methacrylic resin, polyvinyl chloride resin, polyvinylidene chloride resin, olefin resin, polyester resin, styrene resin, urethane resin, polyamide resin, their mixtures, etc. Preferred are fluororesin and silicone resin.

The breathable waterproof layer covers nearly the whole of a one-side surface of the deodorizing layer, preferably at least 80% of a one-side surface of the deodorizing layer, more preferably the whole of a one-side surface of the deodorizing layer. Preferably, the breathable waterproof layer and the deodorizing layer are tightly adhered to each other on their surfaces and superimposed with no space therebetween. With that, the laminate may exhibit a stronger elastic restoration power when pushed, as compared with a case that has a space between the layers, and therefore may have a good effect of sucking up excrements.

In addition, since the breathable waterproof layer has a wide area capable of covering nearly the whole of a one-side surface of the deodorizing layer and that of the porous material layer, it may be clogged only partly even when excrements adhere to the breathable waterproof layer; and accordingly, excrements may be readily removed by the excrements sucking action of the invention therefore securing the gas penetration through the deodorizing layer.

(Regarding Porous Material Layer)

The porous material layer 33 in the first embodiment is composed of a polyurethane open-cell foam having a thickness of 2 mm and a water absorption ratio of 40 times. Preferably, the porous material layer has a thickness of from 0.5 to 10 mm, more preferably from 1 to 5 mm. Also preferably, the layer has a water absorption ratio of at least 20 times, more preferably at least 35 times. Having the thickness and the water absorption ratio on the level as above and applying the force, the porous material layer may readily undergo elastic deformation and may be more effective for sucking up the excrements from the breathable waterproof layer. The water absorption ratio as referred to in the invention is determined as follows: A subject to be analyzed is dipped in water for 3 minutes, then taken out, hung for 1 minute to remove excessive water by spontaneous dripping, and then the weight (weight after water absorption) is measured. The water absorption ratio of the sample is computed according to the following equation:

$$\text{Water Absorption Ratio} = [(\text{weight after water absorption} - \text{weight before water absorption})/\text{weight before water absorption}]$$

Preferably, the porous material layer is formed of an elastic material capable of undergoing elastic deformation when pressed with a finger or the like, and capable of being restored to the original condition; and preferably, a sheet material such as an open-cell foam, a three-dimensional spatial fabric or the like is used for the layer.

As the open-cell foam for the porous material layer, usable are polyurethane, acryl, chloroprene rubber, silicone and the like; and as the three-dimensional spatial fabric, usable is a three-dimensional spatial knitted fabric produced by three-dimensionally connecting two fabrics of constituting the surface and the back thereof with a connecting yarn.

The porous material layer covers nearly the whole of a one-side surface of the breathable waterproof layer, preferably at least 80% of a one-side surface of the breathable waterproof layer, more preferably the whole of a one-side surface of the breathable waterproof layer. Preferably, the porous material layer and the breathable waterproof layer are tightly adhered to each other on their surfaces and superimposed with no space therebetween. With that, the laminate may exhibit a stronger elastic restoration power when pushed, as compared with a case that has a space between the layers, and therefore may have a good effect of sucking up excrements.

In addition, the porous material layer has a wide area capable of covering nearly the whole of a one-side surface of the breathable waterproof layer; and accordingly, even when excrements adhere to the breathable waterproof layer, the adhered excrements could be uniformly removed by the excrements sucking action of the invention, therefore securing gas penetration through the deodorizing layer.

(Regarding Cover Layer)

The cover layer 34 in the first embodiment is composed of a vapor-impervious and liquid-impervious polyester film, and has two slit-like through-holes 36 formed therein.

The cover layer is to prevent excrements from penetrating into the inside of the deodorizing filer as much as possible in the initial stage, and is therefore preferably a liquid-impervious one. For it, usable is the same sheet-like material as that for the breathable waterproof layer. Above all, preferred for the cover layer has a smooth surface and is a vapor-impervious and liquid-impervious plastic film. Using this makes it possible to prevent penetration of liquid or semisolid excrements into the filter and makes it possible to retard adhesion of excrements to the surface of the cover layer.

The plastic film for use for the cover layer includes polyesters, polyolefins (polyethylene, polypropylene, olefin copolymer, etc.), polyvinyl chloride, polyvinylidene chloride, polyamides, polyurethanes, etc.

The cover layer may be water-absorbable in some degree so far as a large quantity of excrements may not penetrate through it; and in such a case, preferably, the cover layer has a water absorption ratio of at most 5 times, more preferably a water absorption ratio of at most 1 time.

The surface of the porous material layer opposite to that to face the breathable waterproof layer may be skin-finished to make the surface part thereof impervious to liquid, and the skin-finished part may serve as the cover layer. In this case of skin finish, the cover layer and the porous material layer may be formed of the same material, and the number of the parts necessary to constitute the deodorizing filter may be decreased. Alternatively, the surface of the porous material layer opposite to that to face the breathable waterproof layer may be coated with a synthetic resin to seal up the pores in the surface of the porous material, and the coated part may serve as the cover layer. In that manner, the cover layer may be one integrated with the porous material layer to an inseparable degree, or may be one formed as a different component layer of the above-mentioned plastic film.

The cover layer covers nearly the whole of a one-side surface of the porous material layer, preferably at least 80% of a one-side surface of the porous material layer, more preferably the whole of a one-side surface of the porous material layer. In that manner, the cover layer and the porous material layer are tightly adhered to each other on their surfaces and superimposed with no space therebetween. With that, the laminate may exhibit a stronger elastic restoration power when pushed, as compared with a case that has a space between the layers, and therefore may have a good effect of sucking up excrements as mentioned below.

(Regarding Through-holes of Cover Layer)

Not specifically defined, the through-holes to be formed in the cover layer may be slit-like, dotted or the like; but preferred are slit-like through-holes not having an area. Usually the slit-like through-holes are kept almost shut up, and therefore penetration of liquid excrements through them is extremely difficult.

Preferably, the area of the through-holes is at most 10% of the surface area of the breathable waterproof layer, more preferably at most 5%. When the area of the through-holes is within the range, then the amount of air to flow into the porous material layer during elastic restoration of the porous material layer of the laminate after compression is larger from the side of the breathable waterproof layer than from the side of the through-holes. As a result, the excrements existing around the breathable waterproof layer could be pushed up by the air that flows thereinto from the side of the breathable waterproof layer, therefore making it possible to prevent the clogging of the breathable waterproof layer.

In case where the through-holes formed in the cover layer are slit-like, preferably, the slits are adjacent to the peripheral edge of the cover layer. In the first embodiment, the slit-like through-holes 36 are formed in such a manner that they run toward the outside from the inside region surrounded by the peripheral part of the cover layer and reach the peripheral part thereof, and from one end of the peripheral part of the cover layer, the through-holes run toward the other end of the opposite peripheral part thereof to cross the cover layer. Accordingly, when the laminate is pushed from the side of the cover layer, then the center part of the slit may be blocked up with the finger, but the slit part around the periphery of the cover layer is hardly blocked up with the finger; and therefore, the excrements filled in the porous material layer could be readily discharged out through the slit part around the periphery. In particular, when the surface of the cover layer forms a curved surface as convexed in the center thereby to have a curved cross-section profile as in Fig. 3, then the slits around the periphery of the cover layer are hardly blocked up and therefore the excrements may be readily discharged out through the slits when the laminate is pushed.

(Regarding Laminate)

The deodorizing filter comprises a laminate of, as sequentially superimposed one upon another, a deodorizing layer, a breathable waterproof layer, a porous material layer and a cover layer.

The layers may be merely superimposed one upon another, but are preferably adhered to each other with an adhesive for tight adhesion thereof.

Preferably, the peripheral parts of the laminate are sealed up so that excrements, especially gas may not flow into the laminate through the peripheral parts thereof. As the peripheral parts of the laminate are sealed up, air does not flow into the laminate through the breathable waterproof layer or through the through-holes of the cover layer when the laminate is compressed and when air is led to flow into the porous material layer owing to the elastic restoration of the laminate. In addition, since the through-holes of the cover layer are mostly blocked up with the finger, air may flow into the porous material layer mainly from the side of the breathable waterproof layer. Accordingly, the excrements existing around the breathable waterproof layer may be pushed up by the air that flows into the laminate from the side of the breathable waterproof layer, whereby the breathable waterproof layer may be prevented from being clogged up.

When the peripheral parts of the laminate are sealed up, the peripheral parts of the individual layers may be separately sealed up, the peripheral parts of the three layers of the breathable waterproof layer, the porous material layer and the cover layer are compressed and sealed up all together, the peripheral parts of the laminate itself additionally including the deodorizing layer are compressed and sealed up all together. When the peripheral parts of the laminate are compressed and sealed up all together, the elastic restoration power in compression of the laminate may be higher and the excrements sucking effect of the laminate may be bettered as compared with a case where the peripheral parts of the individual layers are separately sealed up. This is because the tight adhesiveness of the constitutive layers to each other on their surfaces may be enhanced and the power of reaction to be utilized for elastic restoration after compression may be hardly dispersed. In addition, when the peripheral parts of the above-mentioned three layers or the laminate are compressed all together, the surface of the cover layer may form a curved surface therefore producing a structure where the slits near the periphery of the above-mentioned cover layer are hardly blocked up, and accordingly, when the laminate is pushed, the excrements therein may be readily discharged out.

As the method of compressing and sealing up the peripheral parts of the above-mentioned three layers or the laminate all together, for example, there may be mentioned a method of disposing a hot-melt adhesive between the individual layers and hot-pressing the peripheral parts of the layers; a method of adhering a separate component such as a plastic film or the like to the peripheral parts under compression; a method of surrounding the peripheral parts of the laminate with a hard frame by insert-molding, etc. Above all, preferred is the insert-molding method of surrounding the peripheral parts of the laminate with a frame harder than the laminate. According to the method, the laminate can be fixed in a predetermined position, and therefore the laminate can be readily pushed with a finger and can readily undergo elastic restoration. The hard frame 35 in the first embodiment is formed of a thermoplastic material by insert-molding.

(Regarding Excrements Collector)

The excrements collector comprises a pouch 12 for holding excrements, a gas-discharging vent hole 21 formed in the pouch, and a deodorizing filter 30 that covers the gas-discharging vent hole, in which the deodorizing filter is the above-mentioned deodorizing filter.

The basic constitution of the excrements collector is the same both in the ostomy pouch and in the drainage pouch, comprising a faceplate 11 to be adhered and fixed around the aperture or the like formed in a body surface, a pouch 12 to hold the excrements discharged from the aperture or the like, and an opening 13 through which the faceplate communicates with the pouch.

Regarding the type of the ostomy pouch, the invention is applicable to any of a two-piece type one (two-piece ostomy system) where the faceplate and the pouch are detachable, and a one-piece type one (one-piece ostomy system) where the faceplate and the pouch are integrated. The pouch may be any type of a drain pouch (lower part open type) having a discharge port for excrements and others at the lower part of the pouch, or a closed pouch (lower part closed type) not having such a discharge port.

The pouch and the faceplate may be any conventional ones. The pouch may be one formed by welding two plastic films at the peripheral edges thereof; and for the faceplate, usable is a hydrocolloid adhesive.

The shape of the gas-discharging vent hole 21 to be formed in the pouch is not specifically defined. At least one of U-shaped, V-shaped, S-shaped, slit-like (e.g., linear), circular, oval or the like holes may be formed.

Preferably, the gas-discharging vent hole 21 and the deodorizing filter 30 to cover it are disposed above the opening 13 in an ordinary condition of wearing the excrements collector. This is because, when the deodorizing filter is positioned in the position facing the opening or in the position below the opening, then the deodorizing filter may be often fouled with excrements.

The deodorizing filter may be mounted on the inner surface of the pouch 12 in such a manner that the side of the deodorizing layer 31 of the deodorizing filter 30 faces the gas-discharging vent hole 21, as in the first embodiment; or as in the second embodiment shown in Fig. 4, the deodorizing filter may be mounted on the outer surface of the pouch 12 in such a manner that the side of the cover layer 34 of the deodorizing filter faces the gas-discharging vent hole 21.

EXAMPLES

(Example 1)

An excrements collector of the above-mentioned first embodiment was produced, and human feces were put into the pouch. The feces were made to adhere to the surface of the cover layer of the deodorizing filter by rubbing the outer side of the pouch with fingers, and then with pushing the surface of the cover layer, the feces adhering thereto were wiped off with fingers.

This operation was repeated 100 times, however, the gas venting function of the filter did not almost change from before feces adhesion.

(Comparative Example 1)

Regarding the deodorizing filter of the first embodiment mentioned above, an excrements collector having the same constitution as that of the deodorizing filter of the first embodiment was produced, which, however, did not have the cover layer and the porous material layer. Human feces were put into the pouch. The feces were made to adhere to the surface of the breathable waterproof layer of the deodorizing filter by rubbing the outer side of the pouch with fingers, and then with pushing the surface of the breathable waterproof layer, the feces adhering thereto were wiped off with fingers.

When this operation was repeated 5 times, the deodorizing filter was clogged and its gas venting function greatly worsened.

(Comparative Example 2)

Using the same materials as those for the laminate of the deodorizing filter of Example 1, an excrements collector having substantially the same laminate structure as that of the deodorizing filter disclosed in Fig. 3 to Fig. 6 of the above-mentioned Patent Reference 2 (Japanese Patent 3590084) was produced. Specifically, the deodorizing filter of Comparative Example 2 comprises, as sequentially superimposed one upon another, a deodorizing layer, a breathable waterproof layer, a porous material layer and a cover layer, and is so designed that the peripheral parts of the superimposed layers (breathable waterproof layer, porous material layer, cover layer) are separately adhered to the wall of the pouch, as concentrically surrounding the deodorizing layer (only the cover layer is discontinuously adhered). The deodorizing filter of Comparative Example 2 is so designed that excrements gas penetrates into it through the peripheral parts of the constitutive layers and flows toward the center of the filter, and finally the gas is discharged out of the pouch. Human feces were put into the pouch. The feces were made to adhere to the peripheral part of the cover layer of the deodorizing filter by rubbing the outer side of the pouch with fingers, and then with pushing the surface of the cover layer, the feces adhering to the peripheral edge of the cover layer were wiped off with fingers.

When this operation was repeated 20 to 35 times, the deodorizing filter was clogged and its gas venting function greatly worsened.

**Claims**

1.   A deodorizing filter comprising

      a laminate of, as sequentially superimposed one upon another,
      a deodorizing layer(31),
      a breathable waterproof layer(32) covering a one-side surface of the deodorizing layer,
      a porous material layer (33) covering a one -side surface of the breathable waterproof layer, and
      a cover layer (34) covering a one-side surface of the porous material layer and having through-holes (36),
      wherein gas may pass through the through-holes (36) of the cover layer (34), the porous material layer (33), the breathable waterproof layer (32) and the deodorizing layer (31) in the given order,
      wherein the laminate undergoes elastic deformation when a surface of the laminate is pressed,
      wherein the deodorizing layer (31), the breathable waterproof layer (32), the porous material layer (33), and the cover layer (34) constituting the laminate are tightly adhered to each other with no space between the adjacent layers,
      **characterized in that** the peripheral parts of at le ast three layers of the breathable waterproof layer (32), the porous material layer (33) and the cover layer (34) are sealed up all together under compression so as to protect the laminate from gas penetration through the peripheral parts thereof.

2.   The deodorizing filter according to any one of claim 1, wherein the surface of the cover layer (34) forms a curved surface.

3.   The deodorizing filter according to any one of claim 1 or 2, wherein the peripheral parts of the laminate are surrounded by a frame (35) that is harder than the laminate.

4.   The deodorizing filter according to any one of claims 1 to 3, wherein the porous material layer(33) is formed of an open -cell foam or a three-dimensional spatial fabric.

5.   The deodorizing filter according to any one of claims 1 to 4, wherein the through-holes (36) of the cover layer (34) are slit-like ones.

6.   The deodorizing filter according to claim 5, wherein the slit-like through-holes (36) of the cover layer(34) are so formed as to be adjacent to the peripheral part of the cover layer.

**7.** An excrements collector comprising
a pouch (12) to hold excrements,
a gas-discharging vent hole formed in the pouch (12), and
a deodorizing filter(30) of any one of claims 1 to 6 to cover the gas-discharging vent hole.

**8.** The excrements collector according to claim 7, wherein the deodorizing filter(30) is so mounted on the inner surface of the pouch (12) that the deodorizing layer side of the deodorizing filter (30) faces the gas-discharging vent hole

**9.** The excrements collector according to claim 7, wherein the deodorizing filter(30) is so mounted on the outer surface of the pouch (12) that the cover layer side of the deodorizing filter (30) faces the gas-discharging vent hole.

**Patentansprüche**

**1.** Desodorisierendes Filter umfassend:

ein Laminat aus, eines über dem anderen nacheinander überlagert,
einer desodorisierenden Schicht (31)
einer atmungsaktiven wasserfesten Schicht (32), die die Oberfläche auf einer Seite der desodorisierenden Schicht bedeckt;
einer porösen Materialschicht (33), die die Oberfläche auf einer Seite der atmungsaktiven wasserfesten Schicht bedeckt, und
einer Deckschicht (34), die die Oberfläche auf einer Seite der porösen Materialschicht bedeckt und durchgehende Löcher (36) aufweist,
wobei Gas durch die durchgehenden Löcher (36) der Deckschicht (34), die poröse Materialschicht (33), die atmungsaktive wasserfeste Schicht (32) und die desodorisierende Schicht (31) in der angegebenen Reihenfolge hindurchgehen kann,
wobei das Laminat eine elastische Verformung durchmacht, wenn eine Oberfläche des Laminats eingedrückt wird,
wobei die desodorisierende Schicht (31), die atmungsaktive wasserfeste Schicht (32), die poröse Materialschicht (33) und die Deckschicht (34), die das Laminat bilden, fest aneinander ohne Zwischenraum zwischen den aneinanderliegenden Schichten befestigt sind,
**dadurch gekennzeichnet, dass** die peripheren Teile der mindestens drei Schichten der atmungsaktiven wasserfesten Schicht (32), der porösen Materialschicht (33) und der Deckschicht (34) alle zusammen unter Druck versiegelt werden, um das Laminat gegen Gaspenetration durch die peripheren Teile davon zu schützen.

**2.** Desodorisierendes Filter nach Anspruch 1, wobei die Oberfläche der Deckschicht (34) eine gewölbte Oberfläche bildet.

**3.** Desodorisierendes Filter nach einem der Ansprüche 1 oder 2, wobei die peripheren Teile des Laminats von einem Rahmen (35) umgeben sind, der härter als das Laminat ist.

**4.** Desodorisierendes Filter nach einem der Ansprüche 1 bis 3, wobei die poröse Materialschicht (33) aus einem offenzelligen Schaumstoff oder einem dreidimensional räumlichen Textilstoff gebildet ist.

**5.** Desodorisierendes Filter nach einem der Ansprüche 1 bis 4, wobei die durchgehenden Löcher (36) der Deckschicht (34) schlitzähnliche sind.

**6.** Desodorisierendes Filter nach Anspruch 5, wobei die schlitzähnlichen durchgehenden Löcher (36) der Deckschicht (34) so gebildet sind, dass sie neben dem peripheren Teil der Deckschicht liegen.

**7.** Exkrementauffangbehälter umfassend
einen Beutel (12) zum Halten der Exkremente,
ein Gasabgabe-Entlüftungsloch, das in dem Beutel (12) gebildet ist, und
ein desodorisierendes Filter (30) nach einem der Ansprüche 1 bis 6 zum Bedecken des Gasabgabe-Entlüftungslochs.

**8.** Exkrementauffangbehälter nach Anspruch 7, wobei das desodorisierende Filter (30) so an der Innenfläche des Beutels (12) montiert ist, dass die Seite der desodorisierenden Schicht des desodorisierenden Filters (30) dem Gasabgabe-Entlüftungsloch zugewandt ist.

9.  Exkrementauffangbehälter nach Anspruch 7, wobei das desodorisierende Filter (30) so auf der Außenfläche des Beutels (12) montiert ist, dass die Seite der Bedeckung des desodorisierenden Filters (30) dem Gasabgabe-Entlüftungsloch zugewandt ist.


**Revendications**

1.  Filtre désodorisant, comprenant:

    un stratifié composé, superposées de façon séquentielle les unes au-dessus des autres, de:

    - une couche désodorisante (31),
    - une couche perméable à l'air et étanche à l'eau (32) qui recouvre la surface d'un côté de la couche désodorisante,
    - une couche de matière poreuse (33) qui recouvre la surface d'un côté de la couche perméable à l'air et étanche à l'eau, et
    - une couche de recouvrement (34) qui recouvre la surface d'un côté de la couche de matière poreuse et qui comporte des trous traversants (36),

    dans lequel un gaz peut passer à travers les trous traversants (36) de la couche de recouvrement (34), la couche de matière poreuse (33), la couche perméable à l'air et étanche à l'eau (32) et la couche désodorisante (31), dans l'ordre donné,
    dans lequel le stratifié subit une déformation élastique lorsqu'une surface du stratifié est pressée, et
    dans lequel la couche désodorisante (31), la couche perméable à l'air et étanche à l'eau (32), la couche de matière poreuse (33) et la couche de recouvrement (34) qui constituent le stratifié sont intimement collées les unes aux autres sans aucun espace entre les couches voisines,
    **caractérisé en ce que** les parties périphériques d'au moins trois couches de la couche perméable à l'air et étanche à l'eau (32), de la couche de matière poreuse (33) et de la couche de recouvrement (34) sont scellées les unes aux autres par compression de manière à protéger le stratifié contre toute pénétration de gaz à travers les parties périphériques de celui-ci.

2.  Filtre désodorisant selon la revendication 1, dans lequel la surface de la couche de recouvrement (34) forme une surface courbe.

3.  Filtre désodorisant selon l'une quelconque des revendications 1 ou 2, dans lequel les parties périphériques du stratifié sont entourées par un cadre (35) qui est plus dur que le stratifié.

4.  Filtre désodorisant selon l'une quelconque des revendications 1 à 3, dans lequel la couche de matière poreuse (33) est constituée d'une mousse à cellules ouvertes ou d'un tissu spatial tridimensionnel.

5.  Filtre désodorisant selon l'une quelconque des revendications 1 à 4, dans lequel les trous traversants (36) de la couche de recouvrement (34) sont des trous en forme de fente.

6.  Filtre désodorisant selon la revendication 5, dans lequel les trous traversants en forme de fente (36) de la couche de recouvrement (34) sont formés de manière à être adjacents à la partie périphérique de la couche de recouvrement.

7.  Collecteur d'excréments, comprenant:

    - une poche (12) pour contenir des excréments;
    - un trou d'aération de décharge de gaz qui est formé dans la poche (12); et
    - un filtre désodorisant (30) selon l'une quelconque des revendications 1 à 6 pour recouvrir le trou d'aération de décharge de gaz.

8.  Collecteur d'excréments selon la revendication 7, dans lequel le filtre désodorisant (30) est monté sur la surface intérieure de la poche (12) de telle sorte que le côté de couche désodorisante du filtre désodorisant (30) soit situé en face du trou d'aération de décharge de gaz.

9. Collecteur d'excréments selon la revendication 7, dans lequel le filtre désodorisant (30) est monté sur la surface extérieure de la poche (12) de telle sorte que le côté de couche de recouvrement du filtre désodorisant (30) soit situé en face du trou d'aération de décharge de gaz.

[FIG.1]

(a)

(b)

[FIG.2]

(a)

(b)

20 (inner space of pouch)

[FIG.3]

(a)

(b)

(c)

[FIG.4]

20 (inner space of pouch)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5306264 A
- JP 4218156 A
- JP 3590084 B